# EUROPEAN PATENT SPECIFICATION

(11) **EP 0 620 730 B1**
(45) Date of publication and mention of the grant of the patent: **20.09.2000**
(21) Application number: 93902664.7
(22) Date of filing: 18.12.1992
(51) Int. Cl.: A61K 9/38, A61K 9/50, A61K 9/00

(54) **PROLAMINE COATINGS FOR TASTE-MASKING ORALLY-ADMINISTRABLE MEDICAMENTS**
PROLAMINÜBERZÜGE UM DEN GESCHMACK VON ORAL VERABREICHBAREN MEDIKAMENTEN ZU VERDECKEN
ENROBAGES DE PROLAMINE POUR MASQUER LE GOUT DE MEDICAMENTS S'ADMINISTRANT PAR VOIE ORALE

(30) Priority: 31.12.1991 US 815458
(43) Date of publication of application: 26.10.1994
(73) Proprietor: ABBOTT LABORATORIES, Abbott Park, Illinois 60064-3500 (US)
(72) Inventor: MEYER, Glenn, A., Waukegan, IL 60087 (US); MAZER, Terrence, B., Reynoldsburg, OH 43068 (US)
(74) Representative: Modiano, Guido, Dr.-Ing.
(86) International application number: US9211011
(87) International publication number: WO9312771

(56) References cited:
- EP-A- 0 188 953
- WO-A-85/03000
- WO-A-91/06227
- BE-A- 673 097
- FR-A- 760 403
- FR-A- 1 323 056
- FR-A- 2 361 915
- FR-M- 6 275
- US-A- 3 497 369
- US-A- 3 939 259
- US-A- 4 749 575

## Description

### TECHNICAL FIELD

The present invention relates to oral formulations which effectively mask the undesirable tastes of drugs, such as antibiotics or benzodiazepines, and nutritionals, such as dietary fiber or amino acid supplements, and other similar pharmaceuticals or nutritionals with bitter or otherwise undesirable tastes. In particular; the invention relates to taste-masked medicaments capable of being chewed or swallowed without producing a bitter taste in the mouth.

### BACKGROUND OF THE INVENTION

Oral administration of pharmaceuticals and nutritionals is one of the most popular methods of delivery of such beneficial agents. Chewable or suspension dosage forms are the preferred route of such oral administration for both children and adults who have difficulty swallowing capsules or tablets, with palatability being an extremely important factor in formulating these dosage forms in order to maximize the likelihood that the recipient will take the pharmaceutical or nutritional.

Because of the strong, unpleasant taste of many medicaments, flavorings, either alone or in combination with sweeteners and other additives, have been employed to improve taste and palatabilty. The formulation of a pleasant-tasting and palatable product through the sole use of flavorings, sweeteners and additives, however, has been unsuccessful in many medicaments which have a particularly bitter taste, such as the macrolide family of antibiotics, in particular erythiomycin and clarithromycin. Attempts have been made to formulate these antibiotics into suspension dosage forms or into taste-masked chewable tablets using known coating or encapsulation processes with very limited success.

Tsutomu, in Japanese Patent Application 45-12759, published November 1, 1967, claims pharmaceutical compositions consisting of zein and hydroxypropylmethylcellulose (HPMC) in coating tablets to mask taste and odor. The application employs the high molecular weight polymeric HMPC component (exemplified at levels of 10% and above) for its film-forming properties, in a coating used to prevent the disintegration of Vitamin C as measured by temperature and time.

Pescetti, in U.S. Patent 3,939,259, issued Feb. 17, 1976, employed prolamine from corn grain protein (i.e., zein), with approximately an equal level of shellac and a lesser amount of ethylcellulose, to coat digitoxin particles, but did so to achieve a sustained -release effect. Since the incorporation of ethylcellulose may interfere with absorption of the active agent in a timely manner, its incorporation into the present invention for an imediatately-available medicament would be unsuitable.

Cea, in U.S. Patent 4,384,004, issued on May 17, 1983, discloses the encapsulation of the artificial sweetener, L-aspartyl-L-phenylalanine methyl ester (APM), with additional coating materials, which may include zein, for increasing shelf life stability.

Martin, in U.S. Patent 3,497,369, issued on February 24, 1970, claims a free-flowing powder that, upon the addition of warm water with kneading becomes an insoluble dyeable solid that is moldable for a short time. The powder is zein admixed with a plasticizer, preferably a fatty acid monoester, and optionally a white pigment. There is no suggestion to enclose or coat any active material with the solid.

Benton et al., in Application PCT/US85/00052, (Int'l. Pub. No. WO 85/03000), published 18 July 1985, describe a particulate dual-coated sustained release pharmaceutical dispersed in liquid dosage form having substantial shelf life (35 days) prior to ingestion. The dual coating consists of (1) a lipid such as a fat or moderate melting wax and (2) an overcoat of a cellulose derivative or a prolamine which may include a plasticizer. The coated solid particles are dispersed in a liquid carrier such as high fructose corn syrup and exhibit controlled release in the intestinal tract.

In Belgian patent 673,097 granted to Rhone-Poulenc on 19 September 1966, there is described the taste masking of a bitter substance such as a macrolide antibiotic with a coating of a water soluble vinyl polymer or copolymer, a cellulose ester or ether with free hydroxyl groups, or a prolamine such as zein. The bitter substance is dissolved in a first suitable solvent while the coating material is dissolved in the same solvent or a solvent which is miscible in or dispersible in the first solvent. The solvent phase is then removed by either atomizing the solution or dispersion, producing coated prills, or, by evaporating the solvent phase, leaving a solid which is ground to a particulate form with the antibiotic embedded.

In comparison with these known formulations, the present invention provides a taste masked product in a chewable or nonchewable dosage form or suspension with immediate bioavailability. Additionally, the taste masking ability of the present invention is due solely to the prolamine fraction of grain, with only minor levels of a water insoluble oil or wax of vegetable origin as plasticizer needed to form the film coating.

FR-A-1 323 056 is concerned with taste masking as well as giving a handsome, shiny appearance to pharmaceutical tablets and giving them a protective coating that will stand up during handling and shipping. Two somewhat different coating compositions are applied sequentially, one or more layers of the first, and one or more layers of the second. Each composition contains zein, sodium dioctylsulfosuccinate, polyoxyethylene sorbitan monolaurate and a typically a significant amount of fillers (silica, titanium dioxide, calcium carbonate, talc). A wax coating disclosed in this reference is a final coating used to conserve the polish and fill in surface cavities.

### Summary of the Invention

The present invention is directed to an orally-administrable medicament comprising (a) a core-mixture of a pharmaceutically-active agent or nutritional, having a surface and (b) a single outer polymeric coating layer of from 1 to 35 micrometers thick comprising a prolamine fraction of grain protein, preferably zein or gliadin or mixtures thereof, and a vegetable source water-insoluble oil or wax as plasticizing agent for said prolamine, wherein the ratio of prolamine to plasticizing agent is from 40:1 to 20:3.

The present invention further relates to a medicament wherein the ratio of the pharmaceutically-effective agent or nutritional to the prolamine fraction is 20:1 to 1:1. In particular, the invention comprises taste-masked medicaments capable of being incorporated into a suspension or chewable dosage form without producing a bitter taste.

### Brief Description of the Drawings

Figure 1 is a graph of the plasma concentration of clarithromycin versus time of both the zein-coated clarithromycin suspension and the clarithromycin tablet reference, administered at 125 mg of clarithromycin activity per dog.
Figure 2 is is a graph of the bitterness (on a scale of 0 to 3, where 0 represents no bitterness and a 3 represents a strong bitterness) observed for a clarithromycin concentration in solution.
Figure 3 is a graph of the bitterness observed for the formulation of zein-coated clarithromycin particles in an aqueous environment for both an initially-prepared suspension (Day 0) and one which was prepared and allowed to stand for 7 days, as a function of time after tasting.

### Detailed Description of the Invention:

The present invention relates to the use of an outer polymeric coating layer to effectively taste-mask pharmaceutically-active agents or nutritionals which have bitter or otherwise undesirable tastes. The formulations of the present invention comprise (a) a core of a pharmaceutically-active agent or nutritional, as well as diluents, fillers or other inactive ingredients necessary for the formation of the pharmaceutical core, and (b) a single outer polymeric coating layer of from 1 to 35 micrometers thick comprising a prolamine fraction of grain proteins and a vegetable source water-insoluble oil or wax as plasticizing agent for said prolamine, wherein the ratio of prolamine to plasticizing agent is from 40:1 to 20:3.

The prolamine fraction is purified from corn or wheat and includes zein or gliadin or mixtures thereof, but preferably is zein (the prolamine fraction of corn) which has been purified to between 86-96%, most preferentially, 92-96%. Additionally, the prolamines present in the preferred coating formulation will be present in a solution consisting of 90% food grade ethanol and 10% distilled water at a preferred level of between 5% and 20% prolamine.

The plasticizing agent is a water-insoluble vegetable source oil or wax. The preferred level of coating consists of from 5 % to 100 % of applied film polymeric coating, where the major constituent is the prolamine fraction of gain protein (with the percent representing the weight of film coating relative to the initial weight of the core of the medicament). The most preferred level of coating is between 45 to 75 % weight of coating to weight of the medicament core.

The preparation of the formulation may be accomplished by a variety of coating techniques known in the art, including fluidized bed coating, coacervation (i.e., microencapsulation), or a combination thereof as disclosed by Cea et al. in U.S. Patent 4,384,004. Preferably,wet granulation techniques may be employed to form the medicament core, and fluidized bed coating, with a Wurster column insert may preferentially be employed to apply the coating, as described by Mehta et al. in U.S. Patent 4800087.

The preferred method for preparing the pharmaceutically-active core is to co-mix the active agent with a portion of inactive binder consisting of polyvinylpyrrolidone (Povidone™ made by International Specialty Chemicals Corp.) of a molecular weight range of 30,000 to 400,000 in a weight to weight basis of 5 % to 65 % of the active ingredient. Alternative granulating agents capable of assisting in the formation of a particle containing the active ingredient include, hydroxypropylcellulose (Klucel™, Hercules Corporation), hydroxypropylmethyl cellulose, (Methocel™, Dow Chemical Corp.). Pregelatinized Starch, (Colorcon, Inc.) or any other material suitable for use as a binding agent for the formation of particles capable of being utilized as pharmaceutically active cores. After blending, a sufficient portion of water or food grade ethanol is added to wet mass the mixture into a wet granulation. This material may be either oven-dried under mild heat or dried in a fluidized bed air-drying system, which is capable of performing the task in a more efficient, less time-consuming fashion. The particles are then dried to a specified level of dryness (based on weight loss measurements) and milled to produce a small particle size range. These particles may then be sieved to collect the fraction of particles of a particular size range for subsequent coating. Alternate methods for preparing particles are equally useful at creating particles of a suitable size range.

A wide range of medicaments are suitable for formulation in the present invention. Such medicaments include antibiotics, preferably macrolide antibiotics, and other antibacterial agents, analgesics, antihistamines, decongestants, anti-inflammatory agents, hypnotics, sedatives, tranquilizers, vitamins, enzymes, nutritional supplements, hormones and the like. Those with an especially bitter taste, such as macrolide antibiotics, specifically erythromycin and clarithromycin, are particularly suited for the present invention.

The formulation of the present invention may be incorporated into a variety of pharmaceutical and nutritional products, including pharmaceutical suspensions, pediatric infant formulas, and nutritional supplements.

### Example 1

### A. Preparation of Clarithromycin : Polyvinylpyrrolidone Particles

To a pharmaceutically-active core consisting of clarithromycin, 90%, and polyvinylpyrrolidone (Povidone, K-30, ISP Corp.), 10%, a sufficient amount of food grade ethanol was added with mixing to form a wet mass. The wet granulation was then dried in an oven (between 50° and 60°C) until the loss on drying (LOD) was less than 1%. These particles were then ground to a smaller size and the fraction of particles between 177 and 420 micrometers (microns) collected (40-80 mesh fraction)

### B. Preparation of Zein-Coated Clarithromycin Polyvinylpyrrolidone Particles.

To 4 kg of the Clarithromycin polyvinylpyrrolidone particles, prepared as described above, was applied 2.8 kg of solids contained in a coating formulation consisting of zein (93%) and medium chain triglycerides (7%). This coating formula was dispersed in a mixture of 90% food grade ethanol and 10% distilled water to a level of 10.75% solids (prolamine fraction and medium-chain triglycerides), in this cosolvent mixture. The coating was performed in a Glatt GPCG-5 bottom spray particle coater with a fluidized bed and a Wurster column. Inlet temperature was maintained between 39° and 45°C, with the exhaust temperature between 26° and 29°C, and the atomization pressure on the spray nozzle was maintained between a range of 1.8 x 10⁵ and 2.1 x 10⁵ Pa (26 and 30 pounds per square inch). The flow rate of application of coating liquid to the particles was maintained in the range of 10 to 15 ml per minute.

### Example 2

### Dissolution of Zein-Coated Clarithromycin : Polyvinylpyrrolidone Particles as a Function of pH.

The zein-coated Clarithromycin : polyvinylpyrrolidone particles, prepared as described in Example 1A,were tested using a dissolution apparatus to evaluate the percent of active ingredient released into a 900 ml dissolution bath of pH-buffered solution over a two hour period. A dose of 125 mg of clarithromycin activity was used as a representative dose and dissolution was tested at pH 2.0, 5.0, and 6.8. Samples were withdrawn at 30, 60, 90 and 120 minutes. The results, as shown by Table I, below, indicate no active drug was released at pH 6.8. Rapid release of the active drug was observed at pH 2.0.

**Table 1**

| Dissolution of Zein-Coated Clarithromycin : Polyvinylpyrrolidone Particles as a Function of pH.¹ | | | |
|---|---|---|---|
| Time (minutes) | % Drug Released | | |
| | pH 2 | pH 5 | pH 6.8 |
| 30 | 80.8 (8.1)² | 35.9 (22.3) | nd³ |
| 60 | 90.9 (3.0) | 52.9 (13.2) | nd |
| 90 | 94.7 (4.1) | 62.4 (8.5) | nd |
| 120 | 96.3 (2.8) | 69.4 (11.7) | nd |

| | | | |
|---|---|---|---|
| 1. Particles coated with 70% weight coating to weight of particle. | | | |
| 2. Average (±S.D.), n=3. | | | |
| 3. No detectable response. | | | |

### Example 3

### Release of Zein-Coated Clarithromycin : Polyvinylpyrrolidone Particles as a Function of Time.

To a solution in which sodium bicarbonate (50 mg/5 cm³) was dissolved, 125 mg/5 cm³ of clarithromycin activity (accounting for potency of the active agent and the particles), was added, shaken and observed for release of active agent as a function of time. Samples were withdrawn, filtered, and measured at O,4, 24, 72 and 196 hours. The samples were filtered to remove the suspended particles and the clear filtrate was analyzed for clarithromycin content. The results, as shown in Table 2, below, indicate that very low levels of active drug are released over prolonged periods of time.

**Table 2**

| Release of Zein-Coated Clarithromycin : Polyvinylpyrrolidone Particles as a Function of Time. | | |
|---|---|---|
| Time (hours) | Concentration µg/ml) | Percent % |
| 0 | 20 | 0.08 |
| 4 | 60 | 0.24 |
| 24 | 99 | 0.40 |
| 72 | 118 | 0.47 |
| 196 | 102 | 0.41 |

### Example 4

### Formulation of Zein-Coated Clarithromycin

Zein-coated clarithromycin , comprising 70% coating was formulated as follows:

| Ingredient | Amount per dose (5 cm³) |
|---|---|
| Coated clarithromycin | 260. mg (125 mg activity) |
| Sucrose | 3000. mg |
| Xanthan gum | 7.5 mg |
| Silica Gel | 10.0 mg |
| Potassium Sorbate | 20.0 mg |
| Bubble Gum Flavor | 14.0 mg |
| Sodium Bicarbonate | 50.0 mg |
| Totals: | 3361.5 mg/5 ml final volume |

### Example 5

### Bioavailability of Zein-Coated Clarithromycin

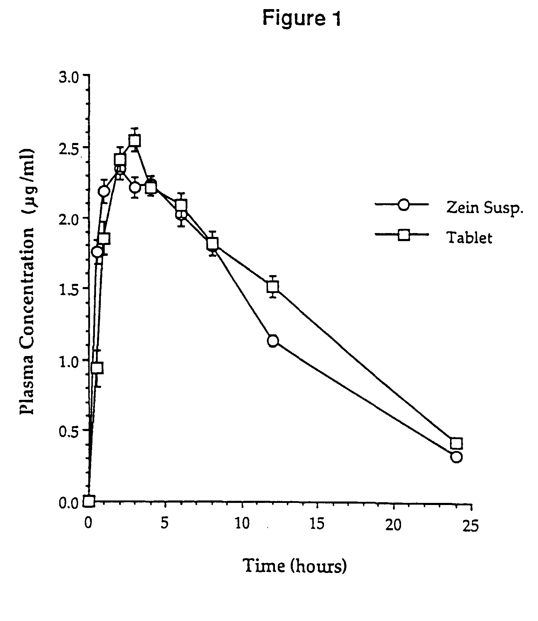

The bioavailability of zein-coated clarithromycin, formulated as described in Example 4, was conducted in a beagle dog model using an single cross-over design. The study compared single dose immediate release tablets containing 125 mg of clarithromycin activity to the microcapsules of clarithromycin prepared as described in Example 1, above, and formulated as described in Example 4, above. The release of clarithromycin was demonstrated by a cross over comparison of the bioavailability of to formula in a beagle dog model compared to a tablet reference containing same. The cross over design allowed the same dogs to receive both the zein-coated clarithromycin suspension as well as to clarithromycin reference tablet of the same dose (125 mg clarithromycin activity). The results, illustrated in Figure 1, demonstrate that to zein-coated clarithromycin suspension did not release the clarithromycin in the suspension (as shown by a representative suspension in Example 3, Table 2), but rapidly released to active drug in the gastrointestinal tract of the dog for equivalent absorption to that of the reference tablet.

### Example 6

### Flavor Evaluation of Zein-Coated Clarithromycin

Comparative flavor evaluation noting characteristics of bitterness of the particle-coated clarithromycin and clarithromycin in solution was conducted. The formulation used was that described in Example 4, and was compared with clarithromycin in solution as a reference standard for the level of bitterness observed for the samples. The study was conducted using trained flavor specialists who standardize their palate with the use of references which were, in this case, to solutions of clarithromycin. The study consists of swirling a dose of the material, solution or suspended formulation, in the mouth and then ranking bitterness, relative to standards, as a function of time after tasting it. The period of time after tasting is evaluated in the event that particles remain or get lodged in the oral cavity before being removed by salivary secretions.

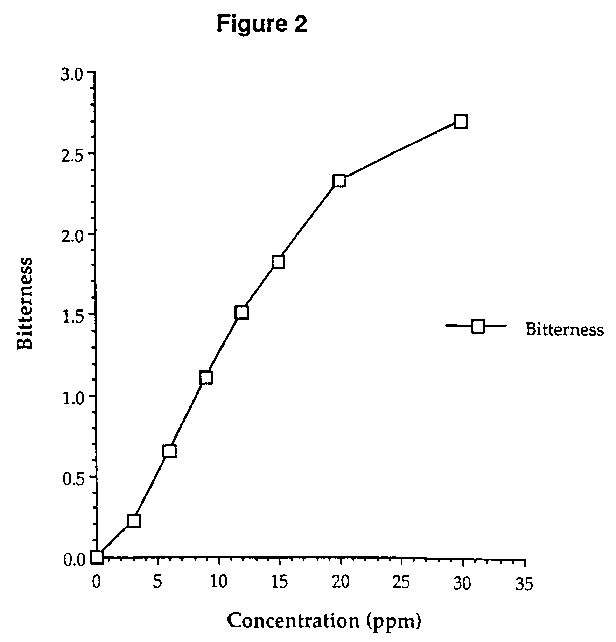

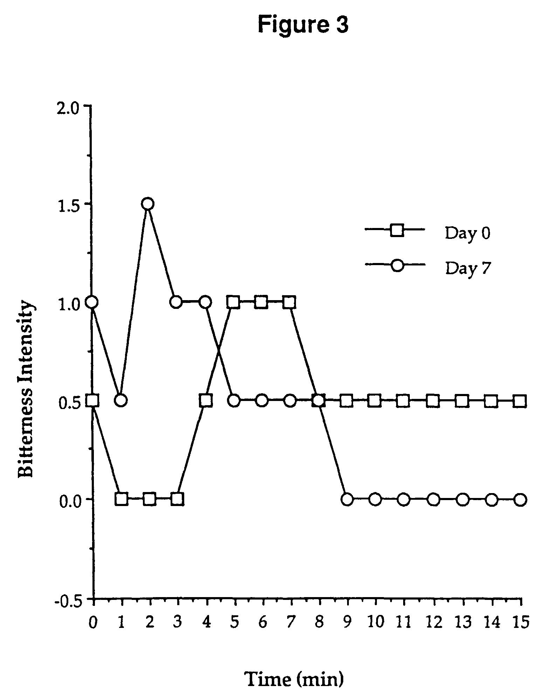

The dose response for the clarithromycin in solution can be seen in Figure 2 and the corresponding level of response for the suspension is shown in Figure 3, as a function of time after tasting, to demonstrate the lack of bitterness of this formulation. Figure 3 thus shows that levels of free clarithromycin available for interaction with taste buds are below 15 ppm even after 7 days in suspension,thus demonstrating the taste-masking ability of the zein coating for an example of a very bitter pharmaceutically-active agent.

## Claims

1. An orally administered medicament consisting of (a) a core of pharmaceutically active agent or nutritional having a surface, and (b) overlaying said surface, a single outer polymeric coating of from 1 to 35 micrometers thick comprising a prolamine fraction of grain proteins and a vegetable source water-insoluble oil or wax as plasticizing agent for said prolamine, wherein the ratio of prolamine to plasticizing agent is from 40:1 to 20:3.

2. A medicament according to claim 1 wherein the ratio of the pharmaceutically active agent or nutritional to the outer polymeric coating is from 20:1 to 1:1.

3. A medicament according to claim 1 wherein the prolamine fraction is zein or gliadin, or a mixture thereof, and said prolamine fraction has been purified to 86-96%.

4. A medicament according to claim 1 which is in the form of a suspension or a chewable dosage form.

5. A medicament according to claim 1 wherein said pharmaceutically active agent or nutritional comprises an antibiotic, vitamin, dietary fiber, analgesic, nutritional, enzyme or hormone.

6. A medicament according to claim 5 wherein said pharmaceutically active agent is a macrolide antibiotic.

7. A medicament according to claim 6 wherein said macrolide antibiotic is clarithromycin or erythromycin.

## Patentansprüche

1. Oral verabreichbares Medikament, das aus (a) einem Kern aus pharmazeutischem Wirkstoff oder Nährstoff mit einer Oberfläche besteht, und (b) eine einzelne äußere Polymerschicht von 1 bis 35 Mikrometern Dicke, die diese Oberfläche überzieht und die eine Prolaminfraktion des Getreideproteins und ein wasserunlösliches Öl oder Wachs pflanzlichen Ursprungs als Weichmacher für das Prolamin enthält, wobei das Verhältnis Prolamin zu Weichmacher von 40:1 bis 20:3 ist.

2. Medikament nach Anspruch 1, wobei das Verhältnis pharmazeutischer Wirkstoff oder Nährstoff zu äußerer Polymerschicht von 20:1 bis 1:1 ist.

3. Medikament nach Anspruch 1, wobei die Prolaminfraktion Zein oder Gliadin oder eine Mischung davon ist, und die Prolaminfraktion auf 86-96% gereinigt ist.

4. Medikament nach Anspruch 1, in Form einer Suspension oder kaubaren Dosierform.

5. Medikament nach Anspruch 1, wobei der pharmazeutische Wirkstoff oder Nährstoff ein Antibiotikum, Vitamin, Ballaststoff, Schmerzmittel, Nährstoff, Enzym oder Hormon enthält.

6. Medikament nach Anspruch 5, wobei der pharmazeutische Wirkstoff ein Makrolidantibiotikum ist.

7. Medikament nach Anspruch 6, wobei das Makrolidantibiotikum Clarithromycin oder Erythromycin ist.

## Revendications

1. Médicament administré par voie orale constitué de (a) un coeur de nutriment ou d'agent à activité pharmaceutique, ayant une surface, et (b) recouvrant ladite surface, un enrobage polymère extérieur unique ayant une épaisseur de 1 à 35 µm comprenant une fraction prolamine de protéines de céréales et une cire ou une huile insoluble dans l'eau de source végétale servant d'agent plastifiant pour ladite prolamine, dans lequel le rapport de la prolamine à l'agent plastifiant est de 40/1 à 20/3.

2. Médicament selon la revendication 1, dans lequel le rapport du nutriment ou de l'agent à activité pharmaceutique à l'enrobage polymère extérieur est de 20/1 à 1/1.

3. Médicament selon la revendication 1, dans lequel la fraction prolamine est la zéine ou la gliadine, ou un de leurs mélanges, et ladite fraction prolamine a été purifiée à 86-96 %.

4. Médicament selon la revendication 1, qui est sous la forme d'une suspension ou d'une forme posologique à croquer.

5. Médicament selon la revendication 1, dans lequel ledit nutriment ou agent à activité pharmaceutique comprend un antibiotique, une vitamine, une fibre alimentaire, un analgésique, un nutriment, une enzyme ou une hormone.

6. Médicament selon la revendication 5, dans lequel ledit agent à activité pharmaceutique est un antibiotique macrolide.

7. Médicament selon la revendication 6, dans lequel ledit antibiotique macrolide est la clarithromycine ou l'érythromycine.
